Europäisches Patentamt

(19) European Patent Office

Office européen des brevets

(11) Publication number: **0 004 465**
A2

(12) **EUROPEAN PATENT APPLICATION**

(21) Application number: 79300466.4

(22) Date of filing: 23.03.79

(51) Int. Cl.²: **B 01 D 53/04,** C 07 C 7/12,
C 01 B 1/35

(30) Priority: 24.03.78 US 889779

(71) Applicant: **AIR PRODUCTS AND CHEMICALS, INC.,
P.O. Box 538, Allentown, Pennsylvania 18105 (US)**

(43) Date of publication of application: 03.10.79
Bulletin 79/20

(72) Inventor: **Hvizdos, Leonard James, 751 Berger Street,
Emmaus Pennsylvania 18049 (US)**

(74) Representative: **Lucas, Brian Ronald, c/o Air Products
Limited Coombe House St. George's Square, New
Malden, Surrey (GB)**

(84) Designated Contracting States: **BE DE FR GB NL**

(54) **Method of regenerating adsorbents.**

(57) Water and carbon dioxide contaminants are removed
from a feed gas stream by adsorption in first and second
adsorbent zones arranged in series. Regeneration of the
adsorbent zones is accomplished by (a) introducing a
heated regeneration gas intermediate the first and second
adsorbent zones and passing the heated regeneration gas
through the first adsorbent zone; and (b) subsequently
passing a low temperature regeneration gas sequentially
through the second and first adsorbent zones.

ACTORUM AG

EP 0 004 465 A2

This invention relates to the regeneration of adsorbents.

During the past few years it has become increasingly common to use adsorbents for removing carbon dioxide and water from gas feed streams. One application is the removal of carbon dioxide and water from air prior to its cryogenic fractionation into oxygen, nitrogen and argon. Other applications include the removal of carbon dioxide and water from natural gas and from hydrogen rich streams.

In all the known applications, the gas feed stream is passed through the adsorbents which remove carbon dioxide and water from the gas. Water is preferentially adsorbed and adsorption is generally continued until carbon dioxide is about to break through the adsorbent. The adsorbent is then regenerated.

Various methods of regeneration have been proposed as, for example described in U.S. Patents 3,150,192; 3,710,547 and 3,738,084 and French Patent 2,005,910.

The aim of the present invention is to provide a new method of regenerating adsorbent which is commercially competitive with the patented techniques of others and, at least in its preferred embodiments, is more thermally efficient than such techniques.

According to the present invention there is provided a method for the regeneration of adsorbents used in the adsorption of water and carbon dioxide contaminants from a feed gas stream passed sequentially through first and second adsorbent zones containing adsorbent wherein the water is adsorbed in the first adsorbent zone and carbon dioxide is adsorbed in the second adsorbent zone, which comprises:

    (a)   introducing a heated regeneration gas stream intermediate the first and second adsorbent zones, and passing the heated regeneration gas through the first adsorbent zone in a direction countercurrent to the flow of the feed gas stream therethrough; and subsequently

(b)   passing cool regeneration gas sequentially through the second and first adsorbent zones in a direction countercurrent to the flow of the feed gas stream therethrough.

Preferably, the adsorbent is a molecular sieve, for example 13X molecular sieve, although other adsorbents, for example activated carbon, could also be used.

Preferably, the regeneration is carried out at a pressure between atmospheric (14.7 psig) and 100 psig. Since adsorption would typically be carried out between 90 and 3000 psig the present invention preferably also includes the step of depressurizing the first and second adsorbent zones preferably through the first adsorbent zone prior to step (a) above.

Advantageously, the heated regeneration gas will be at a temperature between 300$^O$F and 450$^O$F although temperatures outside this range could also be used, for example, between 250$^O$F and 500$^O$F.

Preferably the cool regeneration gas is between 40$^O$F and 130$^O$F although lower temperatures could also be used.

The present invention also provides a method for separating water and carbon dioxide from a feed gas stream which comprises, in sequence:

(a)   passing the feed gas stream at a pressure of from 90 to 3000 psig and a temperature of from 40$^O$F to 120$^O$F sequentially through first and second adsorbent zones containing adsorbent, wherein substantially all of the water is adsorbed in the first adsorbent zone and carbon dioxide is adsorbed in the second adsorbent zone;

(b)   discontinuing the flow of the feed gas stream to the first and second adsorbent zones;

(c)     depressurizing the first and second adsorbent zones through the first adsorbtion zone to a regeneration pressure of between 14.7 and 100 psig;

(d)     introducing heated regeneration gas at a temperature between $300^{O}F$ and $450^{O}F$ intermediate the first and second adsorbent zones, and passing the heated regeneration gas through the first adsorbent zone in a direction countercurrent to the flow of the feed gas stream therethrough; and

(e)     passing cool regeneration gas at a temperature between $40^{O}F$ and $130^{O}F$ sequentially through the second and first adsorbent zones in a direction countercurrent to the flow of the feed gas stream therethrough.

It will be appreciated that during the step of introducing the heated regeneration gas intermediate the first and second adsorbent zones part of the heated regeneration gas could be allowed to flow through the second adsorbent zone. This is not, however, preferred since the heated regeneration gas would tend to push the adsorbed carbon dioxide away from the first adsorbent zone. Therefore, preferably, in both the above methods, during the step of introducing the heated regeneration gas intermediate the first and second adsorbent zones, sufficient cool regeneration gas is passed through the second adsorbent zone in the direction of the first adsorbent zone to inhibit passage of the heated regeneration gas through the second adsorbent zone.

It should be appreciated that the first and second adsorbent zones can be physically separate and distinct or can be formed a single bed of adsorbent separated into said zones by, for example, an inlet for introducing heated regeneration gas.

The feed gas stream can conveniently consist of air although feed gas streams in which the major component is hydrogen or methane can also be used.

For a better understanding of the invention and to show how the same may be carried into effect reference will now be made, by way of example, to the accompanying drawings in which:

Figure 1 is a schematic representation of a preferred embodiment of apparatus for practicing the invention; and

Figures 2 to 7 are diagrams showing the composition of an adsorber at the end of an adsorption cycle and at different times during regeneration.

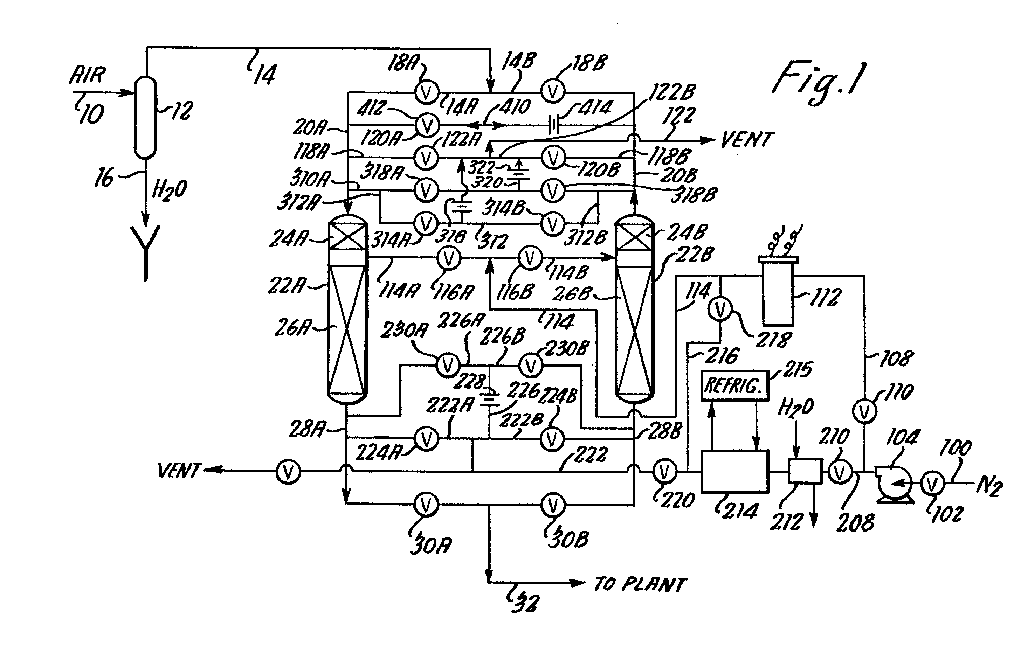

Referring to Figure 1, 7587 lb. moles/hour of moisture laden air at 1950 psia and 40$^O$F are passed through line 10 into separator 12. A certain amount of water leaves the separator 12 through line 16 whilst the saturated air passes into adsorber 22A via lines 14, 14A and 20A and open valve 18A. The adsorber 22A contains two beds 24A and 26A of 13X molecular sieve which retain the remaining moisture and carbon dioxide (400 PPM by volume) in the air whilst allowing the purified air to leave the adsorber 22A through lines 28A and 32 via open valve 30A. The purified air is passed to a cryogenic air separation plant of conventional design (not shown).

As the air passes through the beds 24A and 26A the water is adsorbed preferentially. Adsorbtion is continued until the carbon dioxide is just about to break through bed 26A. At this point, four hours after adsorbtion commences, valves 18A and 30A are closed and valves 18B and 30B are opened thereby ensuring a reasonably uniform supply of purified air through line 32.

Figure 2 shows diagramatically the distribution of water and carbon dioxide in the adsorber at the end of an adsorbtion cycle. (To simplify the diagrams beds 24A and 26A have been drawn as one continuous bed).

In order to regenerate the beds 24A and 26A the pressure in adsorber 22A is first let down to atmospheric. This is achieved by first opening valve 318A which permits the air in the vessel to vent via lines 20A, 310A, 320, orifice 322 and vent 122. The orifice 322 is 0.75 inches in diameter which is sufficiently small to inhibit the molecular sieve leaving the adsorber 22A with the air.

When the pressure in the adsorber 22A reaches 600 psia valve 318A is closed and valve 314A is opened thereby permitting the adsorber 22A to vent through lines 20A, 310A, 312A, orifice 316 and vent 122. The orifice 316 is 1.00 inches in diameter. When the pressure has dropped to 50 psia valve 318A is reopened. As the pressure in the adsorber 22A approaches atmospheric valve 120A is opened thereby permitting the remaining air to vent through lines 118A, 122A and 122. The entire pressure let down takes about 15 minutes.

During pressure let down a small amount of carbon dioxide is desorbed as is a very small quantity of water. The distribution of water and carbon dioxide in the adsorber 22A after pressure let down is shown in Figure 3.

At the end of the pressure let down valves 318A and 314A are closed and valves 116A and 110 opened.

Waste nitrogen, which is substantially free of impurities, and which is obtained from the air separation plant through line 100, passes through valve 102 and is compressed to 19 psia in compressor 104.

The majority of the compressed nitrogen, which leaves the compressor 104 at 130°F then passes through valve 110 and line 108 into electric heater 112 where it is heated to 450°F. The hot nitrogen then passes through lines 114 and 114A via valve 116A. The balance of the nitrogen leaving the compressor 104 passes through line 208 and enters heat exchanger 212 via valve 210. The nitrogen is further cooled in heat exchanger 214 to 40°F and passes through valve 220 and lines 222 and 226 to orifice 228. The orifice 228 restricts the flow of nitrogen which passes through line 226 and valve 230A before entering the bottom of adsorber 22A at

$40^{o}F$. The purpose of introducing nitrogen into the bottom of adsorber 22A is to inhibit hot nitrogen from line 114A passing downwardly through the adsorber 22A and conveying desorbed carbon dioxide to the bottom of the adsorber 22A.

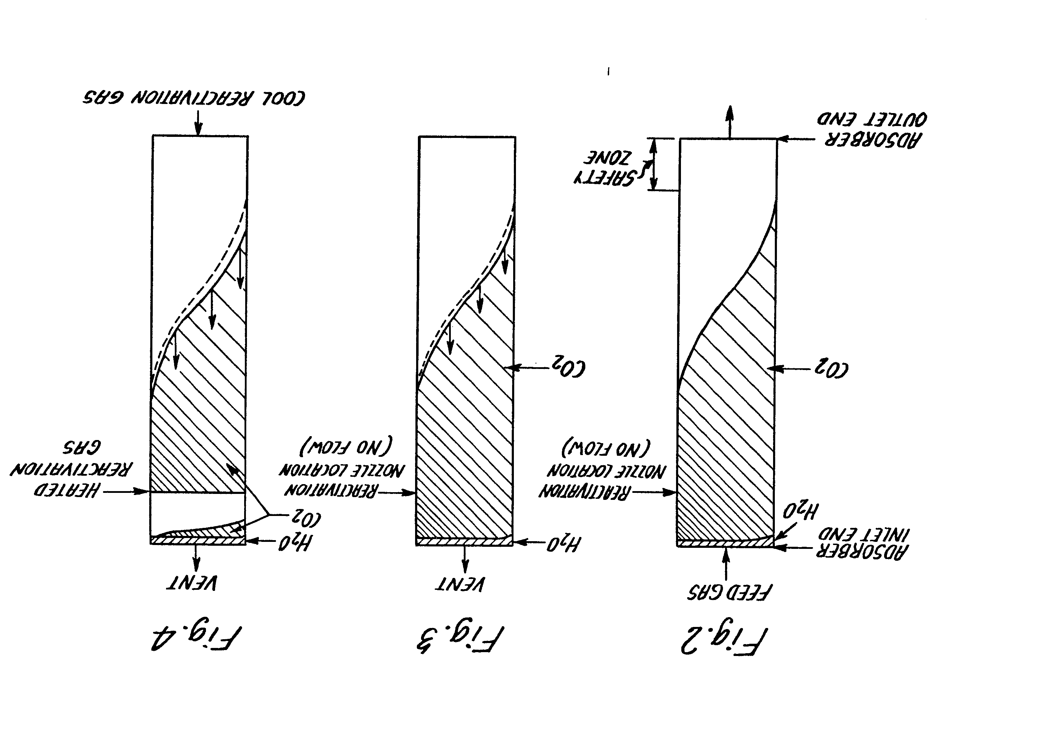

The flow of hot nitrogen through line 114A and through line 226A is continued for 33 minutes after which the lower portion of the bed 24A between the line 114A and the top of the adsorber 22A is at about $450^{o}F$ whilst the temperature in the remainder of the bed 24A decreases to about $40^{o}F$ at the top. As shown in Figure 4, hot nitrogen has desorbed a substantial portion of the carbon dioxide from the lower portion of the bed 24A but little or no water. In this connection the portion of the bed 24A containing carbon dioxide is at less than $100^{o}F$ whilst the water is wholly contained within a zone which, at this stage, is at about $40^{o}F$. It will be noted that a small amount of carbon dioxide has been desorbed from bed 26A.

After the thirty three minutes have elapsed electric heater 112 is turned off but the flow of nitrogen (at $130^{o}F$) is allowed to continue for a further 23 minutes. During this time the heat in the bed 24A is transferred towards the top of the adsorber 22A which rises in temperature to between $150^{o}F$ and $200^{o}F$. All of the carbon dioxide in bed 24A and substantially all the water is desorbed during this time leaving the distribution of the carbon dioxide and water in the adsorber somewhat as shown in Figure 5.

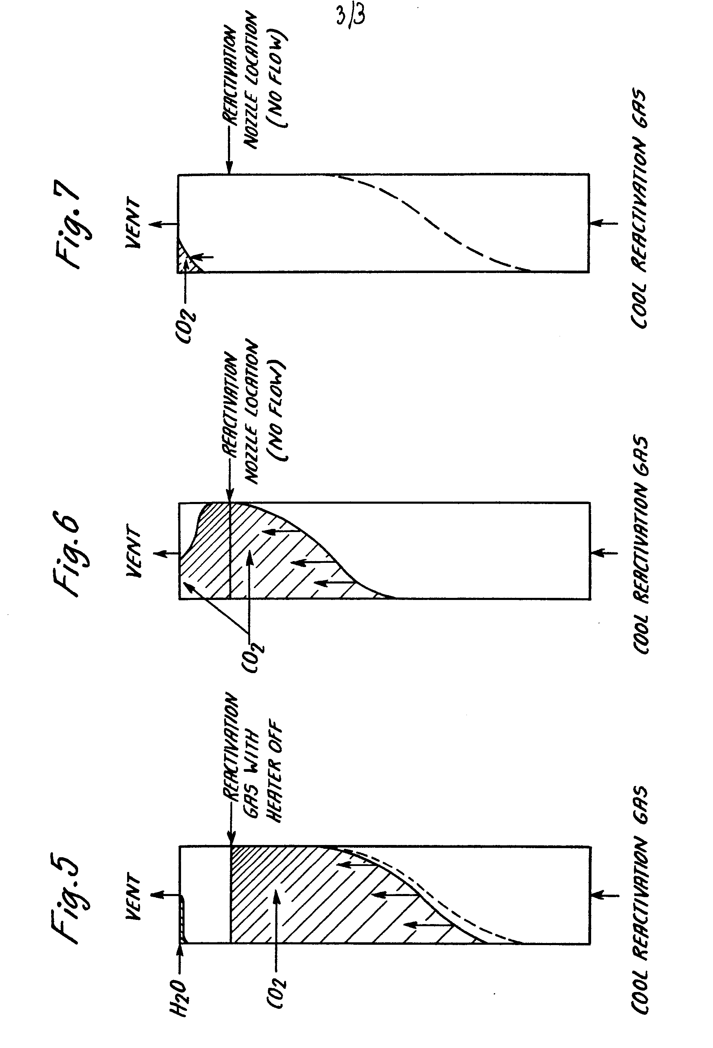

At the end of the 23 minutes valve 224A is opened and valve 230A and 116A are closed. About 4000 lbs/hr of nitrogen leaves refrigerator 214 at $40^{o}F$ and is passed upwardly through adsorber 22A desorbing the carbon dioxide contained in bed 26A and simultaneously cooling bed 24A. Initially little or no carbon dioxide is adsorbed in bed 24A but as its temperature falls a certain amount of carbon dioxide is adsorbed so that after twenty minutes the distribution of the carbon dioxide in the adsorber is somewhat as shown in Figure 6 whereas after a further 32 minutes it is as shown in Figure 7. The carbon dioxide remaining at the end of this period is sufficiently small to be ignored and the adsorber 22A is repressurized by closing valves 224A and 120A and opening valve 412 thereby allowing high pressure air to pass through line 410. Orifice 414 is sized so that the adsorber 22A is repressurized in 15 minutes.

The total regeneration time is 138 minutes.

It will be appreciated that whilst the beds 224A and 26A in adsorber 22A are being regenerated adsorber 22B is on stream adsorbing carbon dioxide and water. When adsorber 22B has completed its adsorbtion cycle, adsorber 22A is placed on stream and the beds 24B and 26B are regenerated. Regeneration is carried out in the manner set out hereinbefore except that the suffix "B" should be used wherever the suffix "A" appears and vice-versa.

The apparatus shown in Figure 1 was then converted for use at low pressure. In particular lines 312, 312A and 312B, together with their associated valves 314A and 314B and orifice 316 were omitted due to the lower pressure.

The upper beds 24A and 24B were each 84 inches in diameter and 12 inches deep and contained 1,200 lbs of 13X molecular sieve. The lower beds 26A and 26B were each 84 inches in diameter and 48 inches deep and contained 5300 lbs each of 1/8 inch 13X molecular sieve.

In use, 2700 lb. moles per hour of saturated air at 134 psia and containing 400 ppm (by volume) carbon dioxide were passed through adsorber 22A for two hours. The flow of air was then diverted to adsorber 22B whilst the molecular sieve in adsorber 22A was regenerated by the following cycle.

Firstly, valve 318 was opened to allow the vessel to depressurize at a rate determined by orifice 322. After the pressure has fallen by a pre-determined amount the valve 120A was opened to vent the remaining air direct to atmosphere. Depressurization was effected over 15 minutes.

Secondly, 9000 lbs per hour of nitrogen heated to approximately 450°F by heater 112 were passed into the adsorber 22A via line 114A. Simultaneously, a small flow of cool nitrogen at 40°F was introduced into the bottom of the adsorber 22A via line 226A. After 35 minutes the heater 112 was turned off but the flow of nitrogen was maintained for a further 11 minutes.

After this further period the flow of nitrogen through line 114A was re-directed via lines 218 and 216 to the bottom of the adsorber 22A for a further 19 minutes. Valve 218 was then closed and the entire flow of nitrogen passed through refrigerator 214 to arrive at adsorber 22A at 40°F. This flow of nitrogen was passed through the adsorber 22A for a further 40 minutes after which the adsorber 22A was repressurized for over a 15 minute period. In this particular case the times of adsorbtion and regeneration were equal.

## CLAIMS

1. A method for the regeneration of adsorbents used in the adsorption of water and carbon dioxide contaminants from a feed gas stream passed sequentially through first and second adsorbent zones containing adsorbent wherein the water is adsorbed in the first adsorbent zone and carbon dioxide is adsorbed in the second adsorbent zone, characterized in that it comprises:

(a) introducing a heated regeneration gas stream intermediate the first and second adsorbent zones, and passing the heated regeneration gas through the first adsorbent zone in a direction countercurrent to the flow of the feed gas stream therethrough; and subsequently

(b) passing cool regeneration gas sequentially through the second and first adsorbent zones in a direction countercurrent to the flow of the feed gas stream therethrough.

2. A method according to Claim 1, wherein the adsorption has been carried on at a high pressure level, characterized in that it further comprising the step of depressurizing the first and second adsorbent zones through the first adsorbent zone prior to step (a).

3. A method according to Claim 1 or 2, characterized in that said heated regeneration gas is at a temperature between $300^\circ F$ and $450^\circ F$.

4. A method according to Claim 1, 2 or 3, characterized in that said cool regeneration gas is between $40^\circ F$ and $130^\circ F$.

5. A method for separating water and carbon dioxide from a feed gas stream characterized in that it comprises, in sequence:

(a) passing the feed gas stream at a pressure of from 90 to 3000 psig and a temperature of from 40°F to 120°F sequentially through first and second adsorbent zones containing adsorbent, wherein substantially all of the water is adsorbed in the first adsorbent zone and carbon dioxide is adsorbed in the second adsorbent zone;

(b) discontinuing the flow of the feed gas stream to the first and second adsorbent zones;

(c) depressurizing the first and second adsorbent zones through the first adsorbtion zone to a regeneration pressure of between 14.7 and 100 psig;

(d) introducing heated regeneration gas at a temperature between 300°F and 450°F intermediate the first and second adsorbent zones, and passing the heated regeneration gas through the first adsorbent zone in a direction countercurrent to the flow of the feed gas stream therethrough; and

(e) passing cool regeneration gas at a temperature between 40°F and 130°F sequentially through the second and first adsorbent zones in a direction countercurrent to the flow of the feed gas stream therethrough.

6. A method according to any preceding Claim, characterized in that during the step of introducing a heated regeneration gas intermediate the first and second adsorbent zones, sufficient cool regeneration gas is passed through the second adsorbent zone in the direction of the first adsorbent zone to inhibit passage of the heated regeneration gas through the second adsorbent zone.

7. A method according to to any preceding Claim, wherein said feed gas stream is air.

8.    A method according to any of Claims 1 to 6, wherein the major component of said feed gas stream is hydrogen or methane.

For the Applicants

*Brian Lucas*

Brian Lucas
CHARTERED PATENT AGENT
EUROPEAN PATENT ATTORNEY

Fig.1

**Fig.2**

FEED GAS

ADSORBER INLET END

H₂O

REACTIVATION NOZZLE LOCATION (NO FLOW)

CO₂

SAFETY ZONE

ADSORBER OUTLET END

**Fig.3**

VENT

H₂O

REACTIVATION NOZZLE LOCATION (NO FLOW)

CO₂

**Fig.4**

VENT

H₂O

CO₂

HEATED REACTIVATION GAS

COOL REACTIVATION GAS

2/3

0004465

# Fig.5

VENT

H₂O →

CO₂ → ← REACTIVATION GAS WITH HEATER OFF

COOL REACTIVATION GAS

# Fig.6

VENT

$CO_2$ ← REACTIVATION NOZZLE LOCATION (NO FLOW)

COOL REACTIVATION GAS

# Fig.7

VENT

$CO_2$ ← REACTIVATION NOZZLE LOCATION (NO FLOW)

COOL REACTIVATION GAS

3/3

0004465